(19) Europäisches Patentamt
European Patent Office
Office européen des brevets

(11) **EP 3 808 837 A1**

(12) **EUROPEAN PATENT APPLICATION**
published in accordance with Art. 153(4) EPC

(43) Date of publication:
**21.04.2021 Bulletin 2021/16**

(51) Int Cl.:
*C12N 5/071* (2010.01)     *C07K 14/76* (2006.01)

(21) Application number: **19820028.9**

(86) International application number:
**PCT/JP2019/023628**

(22) Date of filing: **14.06.2019**

(87) International publication number:
**WO 2019/240255 (19.12.2019 Gazette 2019/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.06.2018   JP 2018114764**

(71) Applicant: **Fuso Pharmaceutical Industries, Ltd.**
**Osaka-shi, Osaka 541-0045 (JP)**

(72) Inventors:
• **YAO, Tatsuma**
  **Osaka-shi, Osaka 536-8523 (JP)**
• **ASAYAMA, Yuta**
  **Osaka-shi, Osaka 536-8523 (JP)**
• **SUGIYAMA, Yui**
  **Osaka-shi, Osaka 536-8523 (JP)**

(74) Representative: **Boult Wade Tennant LLP**
**Salisbury Square House**
**8 Salisbury Square**
**London EC4Y 8AP (GB)**

(54) **CULTURE MEDIUM FOR ASSISTED REPRODUCTION TECHNOLOGY**

(57)    The present application provides a method for an assisted reproductive technology comprising using a medium comprising a low caprylic acid-containing albumin; a medium for said method; and an agent for use in said medium.

EP 3 808 837 A1

**Description**

Technical Field

The present application relates to a medium for an assisted reproductive technology, a method for the assisted reproductive technology using the same, and the like.

Background Art

In recent years, the number of infertile couples has increased every year, partly due to the increasing age at which conception is considered. In the treatment of infertility, fertilization may be assisted depending on the condition. In general, the treatment is stepped up from timed intercourse, ovarian stimulation, artificial insemination to in vitro fertilization / microinsemination.

In vitro fertilization is a method of fertilizing an ovum and a sperm in vitro. Media used for preculture of ova and sperms, insemination, and embryo culture usually contain inorganic salts, energy sources, proteins/macromolecules, and antibiotics. A widely used protein/macromolecule is albumin. Albumin purified from plasma is unstable and easy to form polymers. It is known that the addition of fatty acids is effective for stabilizing the albumin (Non-Patent Document 1, etc.). Among the fatty acids, caprylic acid is commonly used as a stabilizer for albumin (Non-Patent Document 2, etc.).

Microinsemination is a method in which a sperm is injected into an ovum under a microscope to achieve fertilization. Although the fertilization rate by microinsemination is improved compared to in vitro fertilization, a variety of problems have been noted, including lack of a normal fertilization process, physical damage caused by punctures, reduced embryo development rate, and suspected increases in epigenetic diseases. In order to avoid microinsemination, it is desired to improve the fertilization rate in other assisted reproductive technologies. In addition, in order to obtain a good result of fertility treatment, it is desired to improve the fertilization rate in all of the above fertilization methods.

Prior Art Documents

Non-Patent Document

Non-Patent Document 1: Biochim. Biophys. Acta (2004) 1702 (1): 9-17.
Non-Patent Document 2: USP, Albumin Human

Summary of Invention

Technical Problem

The object of the present invention is to provide a method and approach for improving a fertilization rate in an assisted reproductive technology.

Solution to Problem

As a result of studies to solve the above problem, the inventors of the present invention have found that the fertilization rate of in vitro fertilization is improved by using a culture medium prepared by using an albumin obtained by removing caprylic acid from a caprylic acid-added albumin, thereby reaching the present invention. In addition, the present inventors have found that the fertilization rate of in vitro fertilization is improved when various fatty acids other than caprylic acid are added to the caprylic acid-removed albumin as a stabilizer, as compared with the case where the caprylic acid-added albumin is used, thereby reaching the present invention. Furthermore, the inventors of the present invention have found that caprylic acid is specifically removed from the caprylic acid-added albumin by purification with an ion-exchange resin, thereby reaching the present invention.

The present invention provides the following:

[1] An agent for use in a medium for an assisted reproductive technology, comprising a low caprylic acid-containing albumin.
[2] The agent according to [1], wherein the assisted reproductive technology comprises artificial insemination, in vitro fertilization, or microinsemination.
[3] The agent according to [2], wherein the assisted reproductive technology comprises in vitro fertilization.

[4] The agent according to any one of [1] to [3], further comprising a saturated or unsaturated fatty acid having 10 to 20 carbons or a salt thereof.

[5] The agent according to any one of [1] to [4], wherein the saturated or unsaturated fatty acid having 10 to 20 carbons or a salt thereof is selected from the group consisting of lauric acid, myristic acid, pentadecanoic acid, palmitic acid, palmitoleic acid, margaric acid, stearic acid, oleic acid, linoleic acid, a salt thereof, and a mixture thereof.

[6] The agent according to any one of [1] to [5], wherein the low caprylic acid-containing albumin contains 50 $\mu$mol or less of caprylic acid (as a free form) per gram of albumin.

[7] A kit for a medium for an assisted reproductive technology, comprising:

an agent for use in a medium for an assisted reproductive technology, comprising a low caprylic acid-containing albumin; and
an instruction which is that the agent is for use in a medium for an assisted reproductive technology.

[8] A medium for use in an assisted reproductive technology, comprising a low caprylic acid-containing albumin.

[9] The medium according to [8], wherein the assisted reproductive technology comprises artificial insemination, in vitro fertilization, or microinsemination.

[10] The medium according to [9], wherein the assisted reproductive technology comprises in vitro fertilization.

[11] The medium according to any one of [8] to [10], further comprising a saturated or unsaturated fatty acid having 10 to 20 carbons or a salt thereof.

[12] The medium according to any one of [8] to [11], wherein the saturated or unsaturated fatty acid having 10 to 20 carbons or a salt thereof is selected from the group consisting of lauric acid, myristic acid, pentadecanoic acid, palmitic acid, palmitoleic acid, margaric acid, stearic acid, oleic acid, linoleic acid, a salt thereof, and a mixture thereof.

[13] The agent according to any one of [8] to [12], wherein the low caprylic acid-containing albumin contains 50 $\mu$mol or less of caprylic acid (as a free form) per gram of albumin.

[14] A kit for a medium for an assisted reproductive technology, comprising:

a medium for use in an assisted reproductive technology, comprising a low caprylic acid-containing albumin; and
an instruction which is that the medium is for an assisted reproductive technology.

[15] A method for an assisted reproductive technology, comprising using a medium comprising a low caprylic acid-containing albumin.

[16] A method for fertilizing in vitro, comprising using a medium comprising a low caprylic acid-containing albumin.

[17] A method for producing an agent for use in a medium for an assisted reproductive technology in the presence of a low caprylic acid-containing albumin, and a saturated or unsaturated fatty acid having 10 to 20 carbons or a salt thereof.

[18] A method for producing a medium for an assisted reproductive technology in the presence of a low caprylic acid-containing albumin, and a saturated or unsaturated fatty acid having 10 to 20 carbons or a salt thereof.

[19] A method for stabilizing a low caprylic acid-containing albumin for use in an assisted reproductive technology, comrising adding a saturated or unsaturated fatty acid having 10 to 20 carbons or a salt thereof.

[20] A method for removing caprylic acid from a protein, comprising using ion exchange treatment.

[21] The method of according to [20], wherein the protein is albumin.

Effect of Invention

According to the present invention, the fertilization rate in assisted reproductive technology is improved by using a medium comprising a low caprylic acid-containing albumin.

Brief Description of Drawings

Figure 1 shows the in vitro fertilization rate when using human serum albumin (HSA) with caprylic acid removed or re-added. There are significant differences compared to comparative example. (*$p<0.05$, ***$p<0.001$, Chi-squared test, P values adjusted using the Holm procedure.).

Figure 2 shows the in vitro fertilization rate when useing recombinant human albumin (rHA) with caprylic acid removed or re-added. There are significant differences compared to comparative example. (***$p<0.001$, Chi-squared test, P values adjusted using the Holm procedure.).

Figure 3 shows the stability of various albumins after storage at 4 °C or after storage at 37 °C.

Figure 4 shows the in vitro fertilization rate of the use of albumin supplemented with various free fatty acids. There were significant differences compared to the comparative example. (***p<0.001, Chi-squared test, P values adjusted using Holm procedure.)

Figure 5 shows the in vitro fertilization rate and various concentrations of palmitic acid-added albumin. There were significant differences compared to comparative example. (***p<0.001, Chi-squared test, P values adjusted using Holm procedure.).

Figure 6 shows the in vitro fertilization rate and palmitic acid addition concentrations to albumin. There were significant differences compared to comparative example. (***p<0.001, Chi-squared test, P values adjusted by Holm procedure.).

Description of Embodiments

In one aspect, the present application provides an agent for use in a medium for an assisted reproductive technology, comprising a low caprylic acid-containing albumin. The agent of the present application may be used, for example, as a ingredient for the production of media, or as a supplement used as an optional addition to basic culture media for an assisted reproductive technology.

In another aspect, the present application provides a medium for use in an assisted reproductive technology, comprising a low caprylic acid-containing albumin.

In yet another aspect, the present application provides a method for an assisted reproductive technology, comprising using a medium comprising a low caprylic acid-containing albumin.

The term "medium/media" as used herein is not particularly limited as long as it may be applied to gamete(s)/embryo(s), etc. For example, the medium herein may be used to culture gamete(s)/embryo(s), etc., to wash gamete(s)/embryo(s), etc., to suspend gamete(s)/embryo(s), etc., and to preserve gamete(s)/embryo(s), etc.

The term "assisted reproductive technology" as used herein, refers to a method of treatment comprising an artificial manipulation to fertilize gamete(s) (an artificial fertilization), for the purpose of pregnancy. In the assisted reproductive technology as used herein, the artificial fertilization does not always need to be performed, and operations performed in preparation for the artificial fertilization, such as ovum pick-up and cryopreservation of ovum, are also included in the assisted reproductive technology. Examples of the artificial fertilization include artificial insemination, in vitro fertilization, and microinsemination.

In the present application, the subject of assisted reproductive technology is not particularly limited, but is preferably a mammal (humans and non-human mammals (for example, cattle, horses, pigs, dogs, cats, mice, rats, rabbits, and monkeys)), and more preferably a human. Where the subject is a non-human mammal, the assisted reproductive technology may include procedures performed for industrial purposes such as improvement of the production or breeding of the mammal. In addition, studies related to fertilization using human / non-human mammalian gametes may be included in the assisted reproductive technology.

The term "artificial insemination" as used herein, refers to an operation of artificially injecting collected sperm(s) into a female reproductive tract. The collected sperm(s) are usually washed and suspended prior to the injection, and the medium of the present application may also be used as a liquid for such washing and suspension.

The term "in vitro fertilization" as used herein, refers to an operation where an ovum is subjected to an insemination in vitro for fertilization. The medium of the present invention may be used as a medium for the insemination. Usually, the fertilized ovum is then cultured to some extent and the resulting embryo is either implanted in the uterus or cryopreserved. The medium of the present invention may be used as a holding solution for the embryo/fertilized ovum, etc., at the time of implantation into the uterus, or as a freezing liquid for the embryo/fertilized ovum, etc. Usually, sperms with high motility are sorted out from the collected sperms and used for insemination. The medium of the present application may be used for washing, sorting, etc. of the collected sperms. In the assisted reproductive technology comprising in vitro fertilization, the collected ovum and sperm are usually precultured before insemination. The medium of the present application may be used for such preculture.

The term "microinsemination" as used herein, refers to an operation in which a sperm is injected into an ovum under a microscope to cause fertilization. After fertilization, in the same manner as in in vitro fertilization, the fertilized ovum is cultured for a certain period of time, and the embryos/fertilized eggs, etc. are transplanted into the uterus or cryopreserved. The medium of the present invention may also be used in such procedures. In the microinsemination, selection and preculture of sperm and preculture of ovum and the like are usually performed, in the same manner as in in vitro fertilization. The medium of the present application may also be used in such procedures performed before or after microinsemination.

In an assisted reproductive technology, in addition to an artificial fertilization, such as artificial insemination, in vitro fertilization, microinsemination, etc., or in preparation for the artificial fertilization, procedures such as cryopreservation of ovum(s)/sperm(s), in vitro culture of embryo(s), transfer of embryo(s)/blastocyst(s), assisted hatching, cryopreservation of embryo(s), and in vitro culture of ovum are performed in an appropriate combination depending on the patient's condition and symptoms. The medium of the present application may also be used in such procedures performed before and after the artificial fertilization.

In this application, whether the ovum and sperm have been fertilized is determined in the light of common standards in the art. For example, the observation of the second polar body and the male and female pronuclei can be considered fertilization.

In one embodiment, the medium comprising a low caprylic acid-containing albumin of the present application is used before and/or at the time of fertilization in an assisted reproductive technology. Because the medium suitable for fertilization and the medium suitable for embryonic development are generally different, the medium is usually changed after fertilization. The medium comprising a low caprylic acid-containing albumin of the present application may be used before fertilization such as preculture etc., at the time of fertilization, and after fertilization such as embryo development, but is preferably used before fertilization and at the time of fertilization.

In one aspect, the present invention provides a method for fertilizing in vitro, comprising using a medium comprising a low caprylic acid-containing albumin. The method for fertilizing in vitro of the present application may be performed, for example, in fertility treatment or in experiments related to fertilization, etc.

The term "a low caprylic acid-containing albumin" as used herein, means an albumin that does not contain caprylic acid to a degree that adversely affects an assisted reproductive technology (for example, fertilization). The less amount of caprylic acid which may be contained in the low caprylic acid-containing albumin is the more preferable in consideration of the improved fertilization rate.

The amount of caprylic acid contained in the low caprylic acid-containing albumin is not particularly limited as long as it does not adversely affect an assisted reproductive technology (for example, fertilization), but for example, the amount of caprylic acid contained in 1 g of albumin is, as a free form, 50 $\mu$mol or less, preferably 45 $\mu$mol or less, more preferably 40 $\mu$mol or less, still preferably 30 $\mu$mol or less, still more preferably 20 $\mu$mol or less, still more preferably 15 $\mu$mol or less, or still more preferably 10 $\mu$mol or less. Further, examples of the of low caprylic acid-containing albumin include an albumin wherein 0.01 to 50 $\mu$mol, more preferably 0.01 to 45 $\mu$mol, still more preferably 0.01 to 40 $\mu$mol, still more preferably 0.01 to 30 $\mu$mol, still more preferably 0.1 to 20 $\mu$mol, still more preferably 1 to 15 $\mu$mol, or even more preferably 2 to 10 $\mu$mol of caprylic acid (as a free form) is contained relative to 1 g of albumin. The preferred range of caprylic acid (as a free form) contained in 1 g of albumin may be indicated by a combination of a lower limit value selected from 0.01 $\mu$mol, 0.1 $\mu$mol, 1 $\mu$mol, 2 $\mu$mol, and 5 $\mu$mol; and a upper limit value selected from 50 $\mu$mol, 45 $\mu$mol, 40 $\mu$mol, 30 $\mu$mol, 20 $\mu$mol, 15 $\mu$mol, and 10 $\mu$mol.

The method for measuring the amount of albumin is not particularly limited, and may be measured by a method commonly used in the art. For example, the amount of albumin may be measured as the amount of protein, for example, by the Bradford method.

The method for measuring caprylic acid (as a free form) is not particularly limited, and may be measured by a method commonly used in the art. For example, an albumin-containing liquid is extracted (for example, by Bligh-Dyer extraction method), and the resulting lipid fraction is preferably subjected to trimethylsilylation, and then measured by a gas chromatography mass spectrometry.

A preferred method for measuring caprylic acid (as a free form) contained in albumin is exemplified below.

(1) To an albumin is added a solvent (for example, water) to give a mixture wherein, for example, the protein concentration is 5 w/v% by, for example, Bradford method, and the pH of the mixture is opptionally adjusted to, for example, 7 to 8, preferably pH 7.4.

(2) The resulting albumin-containing mixture is subjected to extraction, for example, Bligh-Dyer extraction, and the resulting lipid fraction is subjected to a gas chromatography mass spectrometer (for example, after trimethylsilylation) to measure the amount of free caprylic acid.

In the present invention, the low caprylic acid-containing albumin may be a naturally derived albumin (for example, ovalbumin, porcine albumin, bovine albumin, human albumin) or a recombinant albumin such as bovine type, porcine type or human type. When used for human (including ova, sperms and embryos), human serum albumin (HSA) is preferably used, and more preferably, from the viewpoint of prevention of infectious diseases, recombinant human albumin (rHA) may be used.

In the present application, the method for producing albumin is not particularly limited, and albumin may be produced by a usually used method. For example, albumin may be obtained by purification from plasma, or by purification from products produced in yeast or the like using recombinant techniques. Examples of the purification method include cold ethanol fractionation, a heat treatment method, and a chromatographic purification method.

After the purification (especially, after purification by cold ethanol fractionation / a heat treatment method), the resulting albumin is easily polymerizable and unstable, thus caprylic acid (in a free form or a salt form) may be added thereto. If caprylic acid is added to such an extent as to adversely affect an assisted reproductive technology (for example, fertilization), the low caprylic acid-containing albumin can be obtained by removal of the caprylic acid.

**EP 3 808 837 A1**

In one embodiment, the low caprylic acid-containing albumin used herein may be an albumin obtained as follows: an albumin is obtained through purification by cold ethanol fractionation (for example, Cohn's Fraction V) or a heat treatment method, preferably cold ethanol fractionation. To the obtained albumin is added caprylic acid (a free form or a salt form). The caprylic acid (a free form or a salt form) is removed from the resulting caprylic acid added-albumin to give an low caprylic acid-containing albumin.

Examples of a commercially available albumin include, but are not limited to, the following, which can be used as the low caprylic acid-containing albumin of the present invention after removal of caprylic acid if nessesary.

Recombinant human albumin: CellPrime rAlbumin AF-s(Merck) Albumin derived from human plasma: Human Serum Albumin Solution (Irvine Scientific), HUMAN SERUM ALBUMIN

(InVitroCare)

The method for removing caprylic acid is not particularly limited, and a method usually used for removing fatty acid(s) (for example, a method using activated carbon) can be used. Alternatively, the low caprylic acid-containing albumin of the present application can be obtained by removing the artificially added caprylic acid from an albumin by the method for removing caprylic acid using an ion exchange treatment described below.

In one aspect, the present application provides a method for specifically removing caprylic acid from a protein (for example, albumin) using an ion exchange treatment. For example, biological proteins obtained from living organisms or by fermentation may have residual fatty acids after purification, and the method of the present application may be used to remove caprylic acid from biological proteins. The method of the present application may also be used to remove caprylic acid from proteins to which caprylic acid (a free form or a salt form) has been artificially added.

Although the method of ion exchange treatment used in the method for removing caprylic acid of the present application is not particularly limited, both cation-exchange treatment and anion exchange treatment are preferably performed.

The cation-exchange treatment may be a weakly acidic cation-exchange treatment (for example, wherein carboxylic acid groups are used as exchange groups) or a strongly acidic cation-exchange treatment (for example, wherein sulfonic acid groups are used as exchange groups), and it is preferably a strongly acidic cation-exchange treatment (for example, sulfonic acid groups are used as exchange groups). The ionic form of the exchange group is not particularly limited, and for example, hydrogen, a sodium salt, a potassium salt, or the like, and preferably hydrogen is used. The cation-exchange treatment can be carried out by a usual method using, for example, cation-exchange resins (for example, weakly acidic cation-exchange resins, strongly acidic cation-exchange resins).

The anion exchange treatment may be a weakly basic anion exchange treatment (for example, wherein primary, secondary or tertiary amino groups are used as exchange groups) or a strongly basic anion exchange treatment (for example, wherein quaternary ammonium is used as exchange group), and is preferably a strongly basic anion exchange treatment. Examples of quaternary ammonium include trimethylammonium group and dimethylethanolammonium group, preferably trimethylammonium group. The ionic form of the exchange group is not particularly limited, and for example includes chlorides, hydroxides, acetates, formates, preferably hydroxides are used. The anion exchange treatment can be carried out by a usual method using, for example, anion exchange resins (for example, weakly basic anion exchange resins, strongly basic anion exchange resins).

The support structure of the ion exchange resin is not particularly limited, and a support structure usually used as a support structure of the ion exchange resin (for example, styrenedivinylbenzene) can be used.

As the preferred ion exchange resin, a mixed bed resin containing both a cation exchange resin and an anion exchange resin (for example, AG 501-X8 (D) (BIO-RAD), and AG 501-X8(BIO-RAD)) are preferably used from the viewpoint of workability.

The method for removing caprylic acid of the present invention can be carried out, for example, by mixing ion exchange resin(s) and protein(s) (albumin) which may contain caprylic acid in a suitable solvent (for example, water), (preferably under low temperature (for example, 1 to 10 °C, preferably 2 to 6 °C) and light shielding, for example, 1 to 48 hours, preferably 3 to 36 hours, and more preferably 5 to 30 hours).

The resulting protein-containing liquid may be used as it is, or as a protein-containing agent with low caprylic acid content by optionally performing protein stabilization techniques known in the art such as pH adjustment, addition of stabilizers, etc.

Alternatively, the resulting protein-containing liquid may be concentrated by freeze-drying or ultrafiltration. Filtration may be performed to remove viruses and bacteria. Protein stabilization techniques known in the art may be applied.

The low caprylic acid-containing albumin of the present application may be an albumin to which caprylic acid is not artificially added. For example, the low caprylic acid-containing albumin is an albumin obtained by purification by cold ethanol fractionation (for example, Cohn's Fraction V) or a heat treatment method, preferably cold ethanol fractionation, without artificial addition of caprylic acid after the purification. Since the albumin purified by these purification

6

methods may be unstable, it is preferable to apply stabilization technique(s) appropriately.

Albumin is a protein with a high ability to bind to various substances. For example, albumin derived from serum is bound to various substances contained in serum. Therefore, even if caprylic acid is not artificially added, albumin may carry caprylic acid of biological origin, but may contain it in an amount not adversely affecting an assisted reproductive technology (for example, fertilization). When the amount which can adversely affect an assisted reproductive technology (for example, fertilization) is contained, caprylic acid may be removed by a method for removing caprylic acid using an ion exchange treatment of the present application or a conventional method such as a method using activated carbon, and the resulting albumin may optionally be subjected to stabilization technique(s).

In the present application, the amount of low caprylic acid-containing albumin contained in the medium is not particularly limited, and may be optionally selected within the range of normal use, depending on the purpose of use of the medium in an assisted reproductive technology. For example, the concentration of the low caprylic acid-containing albumin in the medium may be 0.001 to 5 w/v%, preferably 0.01 to 3 w/v%, even more preferably 0.05 to 2 w/v%, and even more preferably 0.1 to 1 w/v%. Further, a preferred range for the concentration of the low caprylic acid-containing albumin contained in the medium may be indicated by a combination of a lower limit selected from 0.001 w/v%, 0.01 w/v%, 0.05 w/v%, and 0.1 w/v%; and a upper limit selected from 5 w/v%, 3 w/v%, 2 w/v%, and 1 w/v%.

In one embodiment of the present application, the agent for use in a medium for an assisted reproductive technology comprising a low caprylic acid-containing albumin of the present application and the medium for use in an assisted reproductive technology comprising a low caprylic acid-containing albumin of the present application may further contain saturated or unsaturated fatty acid(s) having 10 to 20 carbons or salt(s) thereof. The addition of saturated or unsaturated fatty acid(s) having 10 to 20 carbons or salt(s) thereof may improve the effect of assisted reproductive technology (for example, fertilization rate) as compared with the use of caprylic acid-added albumin, and may also stabilize the albumin (for example, prevent the formation of polymers). In the production of the agent or the medium, the low caprylic acid-containing albumin and the fatty acid(s) or salt(s) thereof are preferably closely mixed so that albumin can be stabilized efficiently, and the low caprylic acid-containing albumin and the fatty acid(s) or salt(s) thereof are preferably mixed before being mixed with other ingredients.

In one aspect, the present invention provides a method for stabilizing a low caprylic acid-containing albumin for use in an assisted reproductive technology, comprising an addition of saturated or unsaturated fatty acid(s) having 10 to 20 carbons or salt(s) thereof. By using the method, the albumin may be stabilized (for example, prevention of the formation of polymers), while improving the effect of an assisted reproductive technology (for example, fertilization rate) as compared with the case where an caprylic acid-added albumin is used. In the method, the timing when the fat acid(s) or salt(s) thereof and the low caprylic acid-containing albumin are combined is not particularly limited, as long as that a mixture containing the fatty acid(s) or salt(s) thereof and the low caprylic acid-containing albumin is being formed at the timing of the use of assisted reproductive technology. That is, the fatty acid(s) or salt(s) thereof and the low caprylic acid-containing albumin may be mixed before the assisted reproductive technology, and the fatty acid(s) and the low caprylic acid-containing albumin may be mixed at the time of the assisted reproductive technology. The albumin is preferably stabilized at an early stage, and the fatty acid(s) or salt(s) thereof is preferably added immediately after production of the low caprylic acid-containing albumin.

The saturated or unsaturated fatty acid having 10 to 20 carbons or a salt thereof is not particularly limited as long as it can improve the efficacy of assisted reproductive technology (for example, fertilization rate) and stabilize albumin (for example, prevent the formation of polymers), as compared with the case of using caprylic acid-added albumin. The saturated or unsaturated fatty acid having 10 to 20 carbons or a salt thereof may be one type of fatty acid and/or salt(s) thereof, or a mixture of a plurality of types of fatty acids and/or salts thereof. Preferred examples of the fatty acid(s) or salt(s) thereof include lauric acid, myristic acid, pentadecanoic acid, palmitic acid, palmitoleic acid, margaric acid, stearic acid, oleic acid, linoleic acid, salt(s) thereof, and a mixture thereof, and more preferably, at least palmitic acid or salt(s) thereof is contained. More preferred examples include lauric acid, myristic acid, pentadecanoic acid, palmitic acid, palmitoleic acid, margaric acid, stearic acid, salt(s) thereof, and a mixture thereof. Particularly preferred examples inclide palmitic acid and salt(s) thereof.

The salt of the saturated or unsaturated fatty acid having 10 to 20 carbons is preferably a physiologically acceptable salt. For example, alkali metal salts such as sodium salt and potassium salt; alkaline earth metal salts such as calcium salt, magnesium salt and barium salt; basic amino acid salts such as arginine and lysine; ammonium salts such as ammonium salt and tricyclohexylammonium salt; various alkanolamine salts such as monoethanolamine salt, diethanolamine salt, triethanolamine salt, monoisopropanolamine salt, diisopropanolamine salt and triisopropanolamine salt are preferred. Examples of more preferred salts of the fatty acids of the present invention include alkali metal salts, alkaline earth metal salts, and mixtures thereof.

The amount of the saturated or unsaturated fatty acid(s) having 10 to 20 carbons or salt(s) thereof in the present application is not particularly limited as long as it can improve the efficacy of assisted reproductive technology (for example, fertilization rate) as compared with the case of using caprylic acid-added albumin and stabilize albumin (for example, prevent the formation of polymers). For example, the amount of the fatty acid(s) or salt(s) thereof per 1 g of albumin is, as a free form, 10 to 100 $\mu$mol, preferably 20 to 80 $\mu$mol, more preferably 30 to 50 $\mu$mol. Further, a preferred range for the amount of the fatty acid(s) or salt(s) thereof relative to 1 g of albumin is, as a free form, the range of 5 $\mu$mol to 100 $\mu$mol, and for example, may be indicated in combination with a lower limit selected from 5 $\mu$mol, 10 $\mu$mol, 20 $\mu$mol, and 30 $\mu$mol; and an upper limit selected from 100 $\mu$mol, 90 $\mu$mol, 80 $\mu$mol, 70 $\mu$mol, 60 $\mu$mol, and 50 $\mu$mol.

The form of the agent containing the low caprylic acid-containing albumin for use in the medium for an assisted reproductive technology of the present application is not particularly limited, and may be a liquid form prepared, for example, by mixing with a an appropriate solvent (for example, water, buffers) or the like, whose pH may be adjusted appropriately (for example, pH 7 to 8)); a solid such as freeze-dried; and the like. The agent of the present application may contain additives known as media components, so long as they do not adversely affect assisted reproductive technology (for example, fertilization). Examples include, but are not limited to, inorganic salts (NaCl, KCl, $MgSO_4$, $KH_2PO_4$, $CaCl_2$, $NaHCO_3$, etc.), sugars (for example, glucose, etc.), organic acids (for example, pyruvic acid, lactic acid, etc.), amino acids (for example, L-glutamine, etc.), vitamins (for example, ascorbic acid), antibiotics (for example, gentamicin), and the like. In addition, other additives conventionally used for culture, etc., in an assisted reproductive technology may opptionally be used. The additives are preferably contained within a known concentration range so long as they do not adversely affect fertilization, product stability, etc.

It is preferable that the medium for an assisted reproductive technology comprising a low caprylic acid-containing albumin of the present application contains no ingredient capable of supplying caprylic acid other than the low caprylic acid-containing albumin. The amount of caprylic acid contained in the medium is not particularly limited as long as it does not adversely affect an assisted reproductive technology (for example, fertilization), but for example, the amount of caprylic acid per 1000 g of medium is 2500 $\mu$mol or less, preferably 2200 $\mu$mol or less, more preferably 2000 $\mu$mol or less, still preferably 1800 $\mu$mol or less, still more preferably 1500 $\mu$mol or less, still more preferably 1400 $\mu$mol or less, and still more preferably 1300 $\mu$mol or less as a free form. Further, examples of the amount of caprylic acid (as a free form) contained in 1000 g of the medium include 0.01 to 2500 $\mu$mol, preferably 0.1 to 2200 $\mu$mol, more preferably 0.5 to 2000 $\mu$mol, still more preferably 1 to 1800 $\mu$mol, still more preferably 2 to 1500 $\mu$mol, still more preferably 3 to 1400 $\mu$mol, and even more preferably 5 to 1300 $\mu$mol. Further, the preferable range of the amount of caprylic acid (as a free form) contained in 1000 g of the medium may be indicated by a combination of a lower limit value selected from 0.01 $\mu$mol, 0.1 $\mu$mol, 0.5 $\mu$mol, 1 $\mu$mol, 2 $\mu$mol, 3 $\mu$mol, 4 $\mu$mol, and 5 $\mu$mol; and a upper limit value selected from 2500 $\mu$mol, 2000 $\mu$mol, 1800 $\mu$mol, 1500 $\mu$mol, 1400 $\mu$mol, 1300 $\mu$mol, 1200 $\mu$mol, 1100 $\mu$mol, and 1000 $\mu$mol.

The form of the medium for an assisted reproductive technology comprising a low caprylic acid-containing albumin of the present application is not particularly limited, and may be selected according to the purpose of use of the medium in the assisted reproductive technology, may be, for example, solid medium (such as agar medium, etc.), liquid medium, or powder medium (for example, powder medium which is used as a liquid medium after being dissolved in water etc.) and may be produced by a usual method. As with the agent of the present application, the medium of the present application may contain known additives as components of the medium in a known concentration range so long as they do not adversely affect fertilization. For example, the medium for use in an assisted reproductive technology comprising a low caprylic acid-containing albumin of the present invention can be prepared by adding an low caprylic acid-containing albumin to a known medium for an assisted reproductive technology (for example, HTF medium, KSOM[AA] medium, HFF99 medium, HiGROW IVF medium).

In another aspect, the present application provides a kit that comprises the agent or medium of the present application and an instruction of their use in an assisted reproductive technology. The instruction is not particularly limited in form, and may be a user's manual or may be an access information (URL, QR code, etc.) for browsing the contents of the instruction on the Internet.

Example

The present invention is explained in further detail with reference to Examples and Test examples. However, the scope of the invention is not limited to these Examples.

Test example 1. Removal of caprylic acid by ion-exchange treatment

1.1 Removal processing of caprylic acid

The ion-exchange resin (AG 501-X8 (D), BIO-RAD) previously washed with ultrapure water was added to human serum albumin (HSA, Irvine Scientific) or 10 % recombinant human albumin (rHA, Merck), the mixture was stirred gently with a rotator (RT50, TAITEC) at 4 °C under light shielding for 24 hours. The albumin solution after the treatment was collected, and the protein concentration was determined by the Bradford method, and the concentration and pH were adjusted with ultrapure water and 2 mol/L NaOH to 5 w/v% (pH 7.4).

1.2 Measurement of Free fatty acid

HSA (Example 1) and rHA (Example 2) treated with the ion-exchange resin and the untreated HSA (Comparative example 1) and rHA (Comparative example 2) were extracted by Bligh-Dyer, and the resulting lipid fractions were subjected to trimethylsilylation and gas chromatography-mass spectrometry (7890A GC & 5975C GC/MSD, Agilent) to measure the concentration of free fatty acids.

The results are shown in Table 1.

Table 1. Free Fatty Acid Concentration in 5 w/v% Albumin Solution before and after Ion-Exchange Treatment ($\mu$mol/L)

[Table 1]

| | HSA | | rHA | |
|---|---|---|---|---|
| | Before treatment (Comparative example 1) | After treatment (Example 1) | Before treatment (Comparative example 2) | After treatment (Example 2) |
| Caprylic acid | 4067.3 | 455.3 | 3655.8 | 334.4 |
| Lauric acid | 0.5 | 0.5 | 4.1 | 2.7 |
| Myristic acid | 0.6 | 0.6 | - | - |
| Palmitoleic acid | 0.7 | 0.9 | 12.8 | 12.7 |
| Palmitic acid | 13.6 | 14.1 | 4.0 | 4.4 |
| Linoleic acid | 17.8 | 15.0 | - | - |
| Oleic acid | 4.9 | 4.8 | 3.8 | 4.2 |
| Linolenic acid | 0.9 | 0.9 | - | - |
| Stearic acid | 2.7 | 3.3 | 0.3 | 0.6 |
| Arachidonic acid | 2.4 | 2.8 | - | - |
| Eicosapentaenoic acid | 0.1 | 0.1 | - | - |
| Docosahexaenoic acid | 0.2 | 0.2 | - | - |
| Total | 4112 | 499 | 3681 | 359 |

As shown in Table 1, the ion exchange treatment specifically removed caprylic acid among the free fatty acids contained in the albumin solution.

Test example 2. Inhibiting Effect of caprylic acid on fertilization

2.1 Preparation of Medium

Each of the 4 kinds of 5 w/v% albumin solutions, Comparative example 1, Example 1, Comparative example 2, Example 2, shown in Table 1, as well as albumin solution with 4,000 $\mu$mol/L of sodium caprylate added to Example 1 (Example 1 + 4,000 caprylate) or albumin solutions with 2,000 $\mu$mol/L and 4,000 $\mu$mol/L of sodium caprylate added to Example 2 (Example 2 + 2000 caprylate, Example 2 + 4000 caprylate) were prepared. Those solutions were diluted with HTF medium to give 0.5 w/v% of albumin concentration. 200 $\mu$L of the resulting media were overlaid with mineral oil and allowed to equilibrate overnight at 37 °C under 6 v/v% $CO_2$. The resulting media were used for sperm preculture and in vitro fertilization.

2.2 Collection of Sperm

C57BL/6N male mice (Japan SLC) were sacrificed and the cauda epididymides were excised. Sperm masses obtained by incising the ductus epididymis of the middle of the cauda epididymis were collected in 200 $\mu$L of HTF medium containing 0.1 w/v% polyvinyl alcohol (PVA) under mineral oil. The collected sperm masses were incubated at 37 °C under 6 v/v% $CO_2$ for 10 minutes to disperse homogeneously.

2.3 Preculture of Sperm

The sperms obtained in the above 2.2 were added into the medium prepared in the above 2.1 to give $2 \times 10^6$ sperms/mL and incubated at 37°C under 6 v/v% $CO_2$ for 1 hour until in vitro fertilization.

2.4 Collection of Ovum

To C57BL/6N female mice (Japan SLC), 7.5 IU of PMSG (pregnant mare serum gonadotropin, Aska Pharmaceutical Co., Ltd., Serotropin[tademark]) was intraperitoneally injected, and 48 hours later, 7.5 IU of hCG (Human chorionic gonadotropin, Aska Pharmaceutical Co., Ltd., Gonatropin[Trademark]) was intraperitoneally injected. The mice were sacrificed 15 hours after hCG administration and the oviducts were immediately excised. Ovum-cumulus cell complexes (COCs) were collected from the ampulla of oviduct into the media prepared in the above 2.1. The collected COCs were cultured at 37 °C under 6 v/v% $CO_2$ until in vitro fertilization.

2.5 In vitro fertilization

The sperms prepared in the above 2.3 were added to the medium containing the COCs of the above 2.4 to give a concentration of 100 sperms/$\mu$L of medium. After insemination at 37 °C under 6 v/v% $CO_2$ for 6 hours, ova in which the second polar body and the male and female pronuclei were observed were determined as fertilized ova, and the fertilization rate was calculated by the following equation.

$$\text{Fertilization rate (\%) =} \frac{\text{Number of fertilized ovum}}{\text{Number of ovum with normal morphology}} \times 100$$

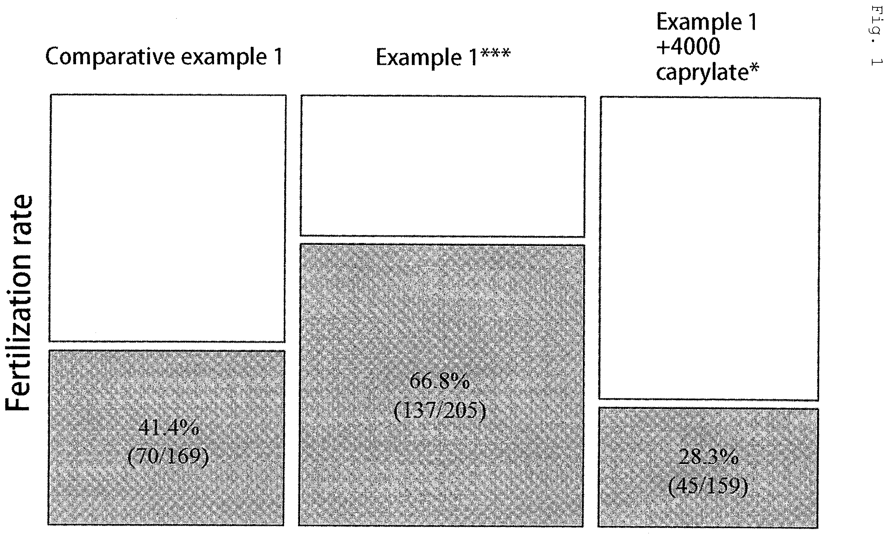

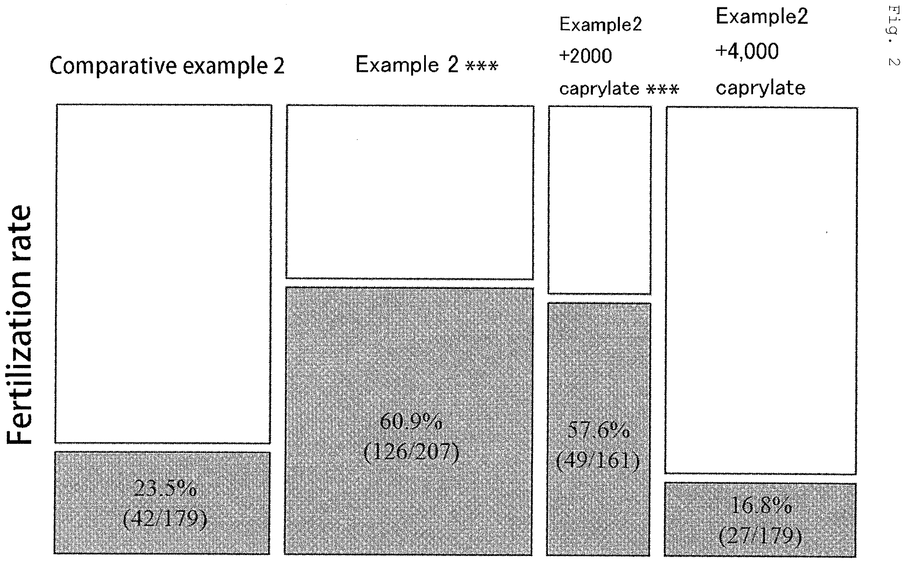

The results are shown in FIGS. 1 and 2.

As shown in FIGS. 1 and 2, the use of the albumin solutions wherein caprylic acid was removed by the ion-exchange treatment improved the fertilization rate, and the use of the albumin solutions re-added caprylate (4,000 $\mu$mol/L) resulted in the reduced fertilization rate. On the other hand, the use of the albumin solution re-added caprylate (2,000 $\mu$mol/L) had no adverse effect on the fertilization rate.
This result shows that the albumin of Examples 1 and 2 which was subjected to the ion exchange treatment is superior to the albumin of Comparative Examples 1 and 2 in that it can bring about the fertility improving effect, and that caprylic acid is involved in the effect.

Test example 3. Stabilization of albumin after removal of caprylic acid

The albumin after removal of caprylic acid was effective in improving the rate of in vitro fertilization. On the other hand, such albumin was unstable and easily polymerized by heating. Fatty acids other than caprylic acid were added to Example 2, and the concentrations at which polymerization could be suppressed under the heating condition were examined.

3.1 Addition of free fatty acid to albumin

Palmitic acid, stearic acid, oleic acid, and linoleic acid were each dissolved in ethanol to each give 1 mg/mL solution. The solutions, 19.2 $\mu$L of palmitic acid solution, 21.3 $\mu$L of stearic acid solution, 21.2 $\mu$L of oleic acid solution, and 21.0 $\mu$L of linoleic acid solution were placed together in one container, and dried by spraying with nitrogen gas. Then, 100 $\mu$L of the albumin solution of Example 2 was added thereto, and the mixture was stirred at 37 °C for 2 hours to give the Example 2 contaninig 4 kinds of free fatty acids (FFA MIX) wherein the total concentration of the free fatty acids was 3000 $\mu$mol/L. The resulting solution was sterilized by filtration through a 0.2 $\mu$m syringe filter, and stored at 4 °C under light shielding until use. In a similar manner, the 4 kinds of free fatty acids (FFA MIX) at the concentrations shown in Table 2 was added to the albumin solution of Example 2.

Table 2. Type and concentration of the free fatty acid added to Example 2

[Table 2]

| | | Albumin solution | Type of fatty acid(s) | Fatty acid concentration ($\mu$mol/L) |
|---|---|---|---|---|
| (1) | | Example 2 | - | - |
| (2) | | Example 2 | FFA MIX | 1000 |
| (3) | | Example 2 | FFA MIX | 1500 |
| (4) | | Example 2 | FFA MIX | 2000 |
| (5) | | Example 2 | FFA MIX | 2500 |
| (6) | | Example 2 | FFA MIX | 3000 |
| (7) | | Example 2 | Caprylic acid | 4000 |
| (8) | | Comparative example 2 | - | - |

3.2 Stability Assessment of Albumin

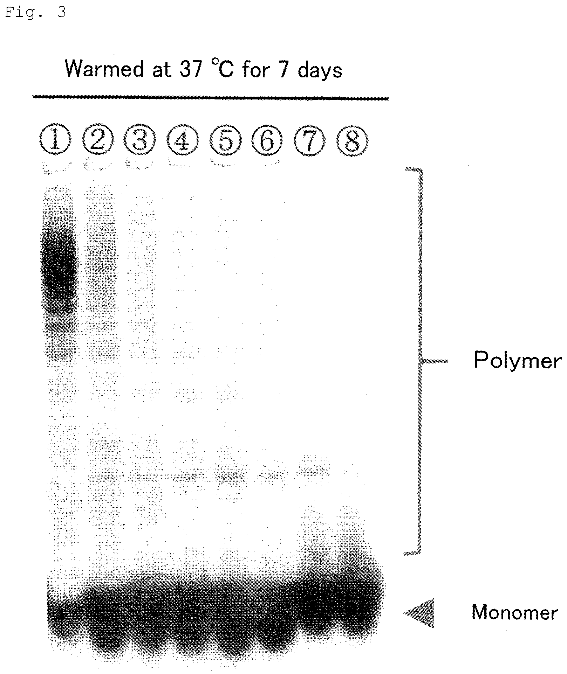

The albumin solutions (1) to (8) shown in Table 2 were stored at 37 °C for 7 days, then diluted to 0.5 w/v%. Each of the diluted solutions (9 $\mu$L) was mixed with 3 $\mu$L of the sample buffer for non-denaturing polyacrylamide gel electrophoresis (Native PAGE). Each of the resulting mixtures (10 $\mu$L) was applied to the 7.5 w/v% gel and subjected to Native PAGE. Protein staining with Coomassie Brilliant Blue was performed to assess the presence or absence of albumin polymerization. The results are shown in Fig. 3.

As shown in FIG. 3, a wide range of the concentrations of free fatty acid(s) suppressed the albumin polymerization.

Test example 4. Fertilization rate of the use of albumin containing various free fatty acid(s)

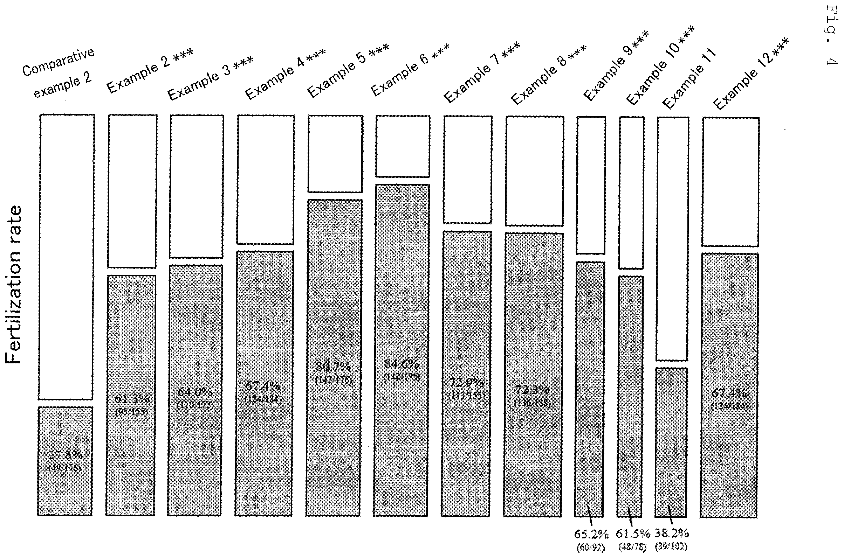

To Example 2, a 5 w/v% albumin solution, was added the free fatty acid(s) as shown in Table 3 (2000 $\mu$mol/L) in the same manner as in Test example 3 to give Examples 3 to 12 as shown in Table 3. The resulting solutions were diluted with HTF medium to give 0.5 w/v% albumin in medium. Using the resulting media, in vitro fertilization tests were performed under the same conditions as Test example 2. The results are shown in FIG. 4.

表 3. Free fatty acids and their concentrations used in the study

[Table 3]

| | Fatty acid | Number of carbon Number of double bond |
|---|---|---|
| Comparative example 2 | Caprylic acid | 8 : 0 |
| Example 2 | - | - |
| Example 3 | Lauric acid | 12 : 0 |
| Example 4 | Myristic acid | 14 : 0 |
| Example 5 | Pentadecanoic acid | 15 : 0 |
| Example 6 | Palmitic acid | 16:0 |
| Example 7 | Palmitoleic acid | 16:1 |
| Example 8 | Margaric acid | 17 : 0 |
| Example 9 | Stearic acid | 18 : 0 |
| Example 10 | Oleic acid | 18 : 1 |

(continued)

|  | Fatty acid | Number of carbon Number of double bond |
|---|---|---|
| Example 11 | Linoleic acid | 18:2 |
| Example 12 | FFA MIX | 16:0, 18:0, 18:1, 18:2 |

As shown in FIG. 4, the use of the albumin containing lauric acid, myristic acid, pentadecanoic acid, palmitic acid, palmitoleic acid, margaric acid, stearic acid, oleic acid, or linoleic acid, and the use of the albumin containing four kinds of fatty acids: palmitic acid, stearic acid, oleic acid, and linoleic acid improved the fertilization rate as compared with the use of Comparative example 2 which comprised a caprylic acid-added albumin.

Test example 5. Concentration of Palmitic acid-added albumin in medium

The used of Example 6 which comprised the palmitic acid-added albumin showed the highest fertilization rate in Test example 4.

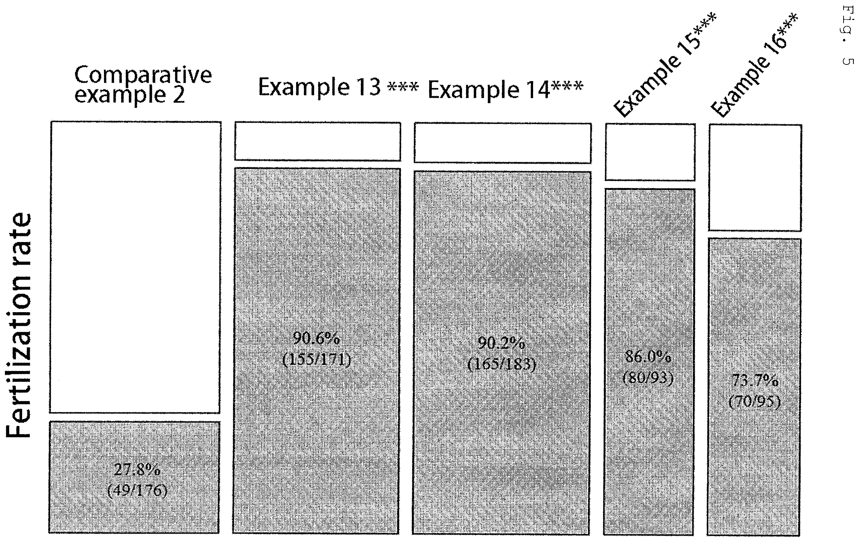

Example 6, a 5 w/v% albumin solution, was diluted with HTF medium to give a 0.5 w/v% albumin medium (10-fold dilution, denoted as Example 6 in Fig. 4), a 0.25 w/v% albumin medium (20-fold dilution, denoted as Example 13 in Fig. 5), a 0.125 w/v% albumin medium (40-fold dilution, denoted as Example 14 in Fig. 5), a 0.0625 w/v% albumin medium (80-fold dilution, denoted as Example 15 in Fig. 5), and a 0.03125 w/v% albumin medium (160-fold dilution, denoted as Example 16 in Fig. 5). Using the resulting media, in vitro fertilization tests were performed under the same conditions as Test example 2. The results are shown in FIG. 5. In FIG. 5, "Comparative example 2" shows a result of the case where Comparative example 2, a 5 w/v% albumin solution, diluted with HTF medium to give a 0.5 w/v% albumin medium. The result of 0.5 w/v% (10-fold dilution, Example 6) is shown in Fig. 4 as Example 6.

As shown in FIGS. 4 and 5, as compared with the use of the albumin of Comparative example 2, Example 6 which was a 5 w/v% albumin solution prepared by addition of palmitic acid (2000 $\mu$mol/L) after the ion exchange treatment showed a higher fertility improving effect at all of the dilutions (0.03125 w/v% to 0.5 w/v% albumin (10 to 160-fold dilution)).

Test example 6. Concentration of palmitic acid added to albumin

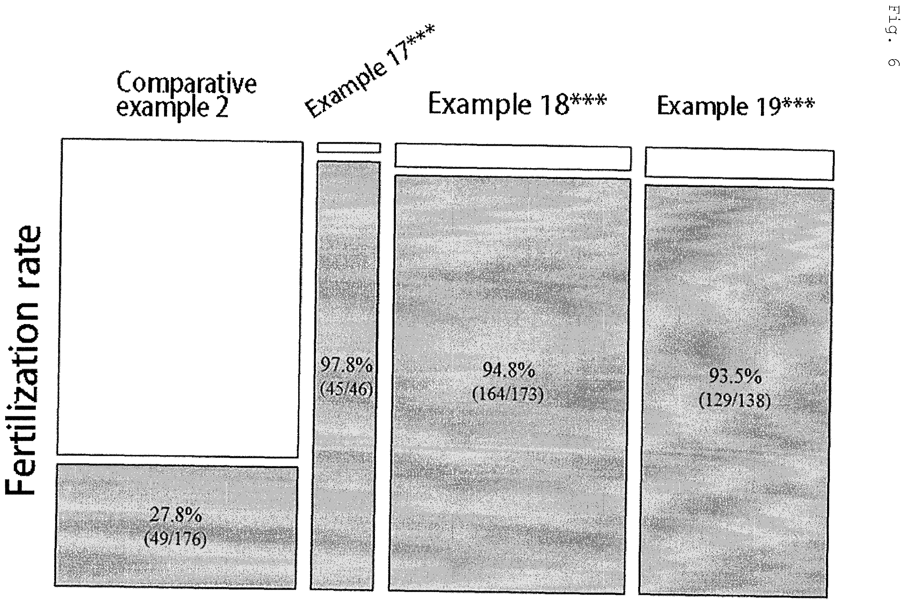

Palmitic acid (4000 $\mu$mol/L) was added to Example 2, wherein the palmitic acid concentration in the 5 w/v% albumin solution was 2 times of Example 6. The resulting solution was diluted with HTF medium to give a 0.5 w/v% albumin medium (10-fold dilution, denoted as Example 17 in Fig. 6), a 0.25 w/v% albumin medium (20-fold dilution, denoted as Example 18 in Fig. 6), a 0.125 w/v% albumin medium (40-fold dilution, denoted as Example 19 in Fig. 6). Using the resulting media, in vitro fertilization tests were performed under the same conditions as Test example 2. In FIG. 6, "Comparative example 2" shows a result of the case where Comparative example 2, a 5 w/v% albumin solution, diluted with HTF medium to give a 0.5 w/v% albumin medium.

As shown in FIG. 6, as compared with the case of the albumin of Comparative example 2, the use of the 5 w/v% albumin solution prepared by addition of palmitic acid (4000 $\mu$mol/L) after the ion exchange treatment had a higher fertility improving effect at all of the dilutions (0.125 w/v% to 0.5 w/v% albumin (10 to 40-fold dilution)).

Industrial Applicability

The use of the agent or medium of the present application can improve the fertilization rate.

**Claims**

1. An agent for use in a medium for an assisted reproductive technology, comprising a low caprylic acid-containing albumin.

2. The agent according to Claim 1, wherein the assisted reproductive technology comprises artificial insemination, in vitro fertilization, or microinsemination.

3. The agent according to Claim 2, wherein the assisted reproductive technology comprises in vitro fertilization.

4. The agent according to any one of Claims 1 to 3, further comprising a saturated or unsaturated fatty acid having 10 to 20 carbons or a salt thereof.

5. The agent according to any one of Claims 1 to 4, wherein the saturated or unsaturated fatty acid having 10 to 20 carbons or a salt thereof is selected from the group consisting of lauric acid, myristic acid, pentadecanoic acid, palmitic acid, palmitoleic acid, margaric acid, stearic acid, oleic acid, linoleic acid, a salt thereof, and a mixture thereof.

6. A kit for a medium for an assisted reproductive technology, comprising:

   an agent for use in a medium for an assisted reproductive technology, comprising a low caprylic acid-containing albumin; and
   an instruction which is that the agent is for use in a medium for an assisted reproductive technology.

7. A medium for use in an assisted reproductive technology, comprising a low caprylic acid-containing albumin.

8. The medium according to Claim 7, wherein the assisted reproductive technology comprises artificial insemination, in vitro fertilization, or microinsemination.

9. The medium according to Claim 8, wherein the assisted reproductive technology comprises in vitro fertilization.

10. The medium according to any one of Claims 7 to 9, further comprising a saturated or unsaturated fatty acid having 10 to 20 carbons or a salt thereof.

11. The medium according to any one of Claims 7 to 10, wherein the saturated or unsaturated fatty acid having 10 to 20 carbons or a salt thereof is selected from the group consisting of lauric acid, myristic acid, pentadecanoic acid, palmitic acid, palmitoleic acid, margaric acid, stearic acid, oleic acid, linoleic acid, a salt thereof, and a mixture thereof.

12. A kit for a medium for an assisted reproductive technology, comprising:

   a medium for use in an assisted reproductive technology, comprising a low caprylic acid-containing albumin; and
   an instruction which is that the medium is for an assisted reproductive technology.

13. A method for an assisted reproductive technology, comprising using a medium comprising a low caprylic acid-containing albumin.

14. A method for fertilizing in vitro, comprising using a medium comprising a low caprylic acid-containing albumin.

15. A method for producing an agent for use in a medium for an assisted reproductive technology in the presence of a low caprylic acid-containing albumin, and a saturated or unsaturated fatty acid having 10 to 20 carbons or a salt thereof.

16. A method for producing a medium for an assisted reproductive technology in the presence of a low caprylic acid-containing albumin, and a saturated or unsaturated fatty acid having 10 to 20 carbons or a salt thereof.

17. A method for stabilizing a low caprylic acid-containing albumin for use in an assisted reproductive technology, comrising adding a saturated or unsaturated fatty acid having 10 to 20 carbons or a salt thereof.

18. A method for removing caprylic acid from a protein, comprising using ion exchange treatment.

19. The method of according to Claim 18, wherein the protein is albumin.

Fig. 1

EP 3 808 837 A1

Fig. 2

EP 3 808 837 A1

Comparative example 2     Example 2 ***     Example2 +2000 caprylate ***     Example2 +4,000 caprylate

Fertilization rate

60.9% (126/207)

57.6% (49/161)

23.5% (42/179)

16.8% (27/179)

Fig. 3

Warmed at 37 °C for 7 days

① ② ③ ④ ⑤ ⑥ ⑦ ⑧

Polymer

Monomer

Fig. 4

Fig. 5

Fig. 6

Fertilization rate

Comparative example 2

Example 17***

Example 18***

Example 19***

27.8% (49/176)

97.8% (45/46)

94.8% (164/173)

93.5% (129/138)

## INTERNATIONAL SEARCH REPORT

| | |
|---|---|
| | International application No. |
| | PCT/JP2019/023628 |

A. CLASSIFICATION OF SUBJECT MATTER

Int. Cl. C12N5/071(2010.01)i, C07K14/76(2006.01)i

According to International Patent Classification (IPC) or to both national classification and IPC

B. FIELDS SEARCHED

Minimum documentation searched (classification system followed by classification symbols)

Int. Cl. C12N5/071, C07K14/76

Documentation searched other than minimum documentation to the extent that such documents are included in the fields searched

```
Published examined utility model applications of Japan      1922-1996
Published unexamined utility model applications of Japan    1971-2019
Registered utility model specifications of Japan            1996-2019
Published registered utility model applications of Japan    1994-2019
```

Electronic data base consulted during the international search (name of data base and, where practicable, search terms used)

JSTPlus/JMEDPlus/JST7580 (JDreamIII), CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | 林茂空ら，体外受精培養液中のオクタン酸濃度と胚発生への影響, 日本生殖医学会雑誌, 2016, vol. 61, no. 4, p. 340, O-016, non-official translation (HAYASHI, Shigehiro et al. Octanoic Acid Density in In-Vitro Fertilization Culture Solutions and Effects on Embryogenesis. Journal of Japan Society for Reproductive Medicine.) | 1-16<br>17 |
| X<br>Y | JP 2014-520535 A (NOVOZYMES BIOPHARMA DK A/S) 25 August 2014, claims, paragraphs [0005], [0077] & US 2014/0234966 A1 & WO 2013/006675 A1, claims, page 2, lines 5-8, page 16, lines 6-10 & EP 2729492 A1 & CN 103649111 A | 1-16<br>17 |

☒ Further documents are listed in the continuation of Box C. ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| | |
|---|---|
| Date of the actual completion of the international search | Date of mailing of the international search report |
| 01.07.2019 | 10.07.2019 |

| | |
|---|---|
| Name and mailing address of the ISA/ | Authorized officer |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/023628 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 4-352727 A (SHIONOGI & CO., LTD.) 07 December 1992, claims, examples (Family: none) | 17 |
| Y | JP 2006-506435 A (TAURUS HSA LLC) 23 February 2006, paragraph [0029] & US 2004/0097710 A1 & WO 2004/046311 A2, paragraph [0047] & EP 1562991 A2 & CN 1732185 A | 17 |
| X | SCHEIDER, W., FULLER, J. K., An effective method for defatting albumin using resin columns, Biochim. Biophys. Acta, 1970, vol. 221, pp. 376-378, page 376, paragraph [0002] | 18-19 |
| X | WO 2014/192938 A1 (AJINOMOTO CO., INC.) 04 December 2014, claims, paragraph [0028] & US 2016/0075993 A, claims, paragraph [0092] & EP 3006558 A1 & CN 105283541 A & KR 10-2016-0012224 A | 18-19 |
| A | JP 2015-506673 A (FACTOR BIOSCIENCE INC.) 05 March 2015, entire text & US 2013/0029418 A1 & WO 2013/086008 A1 & EP 2788033 A1 & CN 104080482 A & KR 10-2014-0109925 A | 1-19 |
| A | JP 2015-509381 A (JANSSEN BIOTECH, INC.) 30 March 2015, entire text & US 2013/0236973 A1 & WO 2013/134378 A1 & EP 2823037 A1 & CN 104160018 A & KR 10-2014-0131999 A | 1-19 |
| A | QUINN, P., and WHITTINGHAM, D. G., Effect of fatty acids on fertilization and development of mouse embryos in vitro, J. Androl., 1982, vol. 3, pp. 440-444 | 1-19 |
| A | FREDRICKSON, J. et al., The impact of the protein stabilizer octanoic acid on embryonic development and fetal growth in a murine model, J. Assist. Reprod. Genet., 2015, vol. 32, pp. 1517-1524 | 1-19 |
| A | LEONARD, P. H. et al., Variability in protein quality used for embryo culture: embryotoxicity of the stabilizer octanoic acid, Fertil. Stern., 2013, vol. 100, no. 2, pp. 544-549 | 1-19 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Biochim. Biophys. Acta,* 2004, vol. 1702 (1), 9-17